# EUROPÄISCHE PATENTSCHRIFT

(11) **EP 2 347 784 B1**
(45) Veröffentlichungstag und Bekanntmachung des Hinweises auf die Patenterteilung: **05.03.2014**
(21) Anmeldenummer: 10182062.9
(22) Anmeldetag: 16.10.2001
(51) Int. Cl.: A61M 16/06, A61M 16/08

(54) **Atemmaske zur Zufuhr eines Atemgases zu einem Maskenanwender**
Breathing mask for supplying breathing gas to a mask user
Masque respiratoire fournissant un gaz respiratoire vers l'utilisateur du masque

(30) Priorität: 19.10.2000 DE 20017940 U; 19.10.2000 DE 10051891
(43) Veröffentlichungstag der Anmeldung: 27.07.2011
(62) Teilanmeldung aus: 01987682.0
(73) Patentinhaber: ResMed R&D Germany GmbH, 82152 Martinsried (DE)
(72) Erfinder: Biener, Achim, 85445 Aufkirchen (DE); Lang, Bernd, 82166 Gräfelfing (DE)
(74) Vertreter: Vossius & Partner

(56) Entgegenhaltungen:
- EP-A2- 1 099 452
- WO-A1-00/20072
- WO-A1-99/43375
- DE-A1- 3 707 952
- DE-A1- 19 808 105
- US-A- 5 243 971

## Beschreibung

Die Erfindung betrifft eine Atemmaske zur Zufuhr eines Atemgases zu einem Maskenanwender sowie eine Ableitungseinrichtung zur Ableitung von Atemgas.

Derartige Atemmasken finden Anwendung insbesondere im medizinischen Bereich beispielsweise im Bereich der Schlafmedizin zur Behandlung schlafbezogener Atmungsstörungen.

Üblicherweise umfassen diese Masken eine Dichtlippeneinrichtung zur Abdichtung einer Maskenauflagezone gegenüber der Umgebung. Bei Nasenmasken erstreckt sich die Maskenauflagezone vom Oberlippenbereich in den, den Nasenflügeln des Maskenanwenders benachbarten Gesichtsbereich und von hieraus in den Bereich des Nasenrückens. Die Dichtlippeneinrichtung ist üblicherweise aus einem elastomeren Material gebildet und über eine Kopfbandanordung unter einer vorgegebenen Andruckkraft gegen die Maskenauflagezone gedrängt.

Insbesondere bei der Langzeitanwendung derartiger Atemmasken besteht das Problem, daß die geforderte Dichtwirkung nur unter vergleichsweise hohen Maskenanpreßkräften erreicht werden kann. Durch die erforderlichen hohen Maskenanpreßkräfte wird der Tragekomfort beeinträchtigt. Zudem besteht die Gefahr, daß im Bereich der Maskenauflagezone Druckstellen entstehen.

DE 19808105 A1 offenbart ein Verfahren zur Herstellung einer Individual-Atemmaske und eine nach dem Verfahren hergestellte Atemmaske.

US 5,243,971 A offenbart eine nasale Maske zum Gebrauch in einem CPAP-System.

Der Erfindung liegt die Aufgabe zugrunde, eine Atemmaske zur Zufuhr eines Atemgases zu schaffen, die sich bei ausreichend hoher Dichtigkeit durch einen hohen Anwendungskomfort auszeichnet.

Die Erfindung wird durch den Gegenstand des unabhängigen Anspruchs definiert. Bevorzugte Ausführungsformen sind in den abhängigen Ansprüchen wiedergegeben.

Eine Atemmaske zur Zufuhr eines Atemgases zu einem Maskenanwender mit einer Dichtlippeneinrichtung zur Abdichtung einer Maskenauflagezone, einem Maskenbasiskörper zur Bildung eines Maskeninnenraumes und einer Anschlußeinrichtung zum Anschluß wenigstens einer Atemgasleitung, wobei der Maskenbasiskörper und/oder die Anschlußeinrichtung mit wenigstens einer aus einem elastomeren Material gebildeten Entkoppelungsstruktur versehen sind, die eine Relativbewegung zwischen einem sich an den Maskenbasiskörper anschließenden Umfangsfußbereich der Dichtlippeneinrichtung und der Atemgasleitung in einem Kippwinkelbereich von wenigstens 5° zuläßt, wird diskutiert.

Dadurch wird es auf vorteilhafte Weise möglich, eine ausreichende Dichtwirkung bei verminderter Maskenhaltekraft auf zuverlässige Weise zu erreichen, da in vorteilhafter Weise selbst bei Bewegung des Kopfes des Maskenanwenders keine Kräfte oder Momente über die Atemgasleitung auf die Dichtlippeneinrichtung übertragen werden können. In weiterhin vorteilhafter Weise wird auch eine Relativbewegung zwischen der auf dem Gesicht des Maskenanwenders aufsitzenden Dichtlippeneinrichtung sowie einer vorzugsweise, (z.B durch einen an einem Versteifungsrahmen gebildeten Schlauchhaltebügel) stirnseitig fixierten Atemgasleitung möglich.

Die Entkoppelungsstruktur ist gemäß einer diskutierten Ausführungsform durch eine Falten- oder Rollbalgstruktur gebildet. Diese Falten- oder Rollbalgstruktur ist vorzugsweise unter Verwendung eines Kernelementes integral mit der Dichtlippeneinrichtung ausgebildet.

Gemäß einer diskutieren Ausführungsform ist die Falten- oder Rollbalgstruktur an der Anschlußeinrichtung ausgebildet. Dieser Falten- oder Rollbalgstruktur kann hierbei durch Zonen mit abgestimmten Wandstärken eine Gelenkcharakteristik verliehen werden durch welche sowohl Dreh- bzw. Kippmomente als auch Axialbewegungen der Atemgasleitung nicht zur Einleitung etwaiger Kräfte in die Dichtlippeneinrichtung führen.

Eine ebenfalls diskutiere Ausführungsform ist dadurch gegeben, daß die Entkoppelungsstruktur an dem Maskenbasiskörper ausgebildet ist. Hierdurch wird es möglich, die Übertragung unerwünschter Kräfte auf die Dichtlippeneinrichtung zu vermeiden und zugleich auch etwaige Bewegungen des Maskenbasiskörpers relativ zur Dichtlippeneinrichtung in ausreichendem Maße, abzukoppeln.

Eine im Hinblick auf besonders geringe Betriebsgeräusche vorteilhafte Ausführungsform ist dadurch gegeben, daß im Bereich der Entkoppelungsstruktur wenigstens eine Kanaleinrichtung ausgebildet ist, zur Schaffung einer Verbindung zwischen dem Maskeninnenbereich und der Umgebung.

Vorzugsweise ist die Kanaleinrichtung durch wenigstens eine Durchgangsöffnung gebildet. Die Durchgangsöffnung weist vorzugsweise einen Querschnitt auf der auf eine vorgegebene Druck/Votumenstromcharakteristik abgestimmt ist.

Eine besonders geräuscharme Ableitung kann dadurch erreicht werden, daß die Kanaleinrichtung sich hinsichtlich ihres Querschnittes in Strömungsrichtung kontinuierlich oder stufenweise verjüngt.

Eine ebenfalls im Hinblick auf eine geringe Geräuschemission vorteilhafte Ausführungsform ist dadurch gegeben, daß in einem Austrittsmündungsbereich der Kanaleinrichtung eine scharfe Mündungskante ausgebildet ist. Vorzugsweise ist in der Kanaleinrichtung ein Abschnitt engsten Querschnitts definiert, wobei die Länge des Abschnitts engsten Querschnitts kleiner ist als 2 mm. Hierbei ist es in vorteilhafter Weise möglich, die Kanalgeometrie so zu gestalten, daß der engste Querschnitt der Kanaleinrichtung in einem Membranelement ausgebildet ist. Hierdurch wird die Einkoppelung etwaiger Körperschallereignisse in die Maskenstruktur noch weiter reduziert. Der Durchmesser des Membranelementes ist vorzugsweise wenigstens 30% größer als der Durchmesser der darin gebildeten Drosseldurchgangsöffnung.

Die bewegliche Ankoppelung der Dichtlippeneinrichtung erfolgt vorzugsweise indem die Entkoppelungsstruktur sich in dem Maskenbasiskörper in Umfangsrichtung der Dichtlippeneinrichtung entlang eines Übergangsbereiches zwischen einem Fußbereich der Dichtlippeneinrichtung erstreckt und durch wenigstens eine Umfangsfalte oder eine Rollbalgstruktur gebildet ist.

Gemäß einem Aspekt der vorliegenden Erfindung und vorzugsweise in Kombination mit den vorangehend beschriebenen Maßnahmen wird eine auf die individuelle Gesichtstektur des Anwenders abstimmbare Atemmaske geschaffen, die eine Stirnauflageeinrichtung aufweist, zur Abstützung der Maske im Stirn- oder Nasenwurzelbereich der Person, und ein Versteifungselement umfasst, zur Aussteifung der Atemmaske mit einem dem Bereich der Dichtlippeneinrichtung zugeordneten ersten Versteifungsabschnitt und einem der Stirnauflageeinrichtung zugeordneten zweiten Versteifungsabschnitt, wobei die Relativposition der beiden Versteifungsabschnitte zueinander einstellbar veränderbar ist.

Dadurch wird es auf vorteilhafte Weise möglich, eine für die jeweilige Gesichtstektur des Maskenanwenders optimale Flächenpressungsverteilung sowohl im Bereich der Dichtlippenauflagezone, als auch im Stirnbereich zu erreichen.

Eine im Hinblick auf eine einfache Handhabbarkeit vorteilhafte Ausführungsform der Erfindung ist dadurch gegeben, daß die beiden Versteifungsabschnitte über eine Gelenkeinrichtung miteinander gekoppelt sind. Die Gelenkeinrichtung kann hierbei in vorteilhafter Weise durch eine Film-Scharniereinrichtung gebildet sein.

Gemäß einer besonders bevorzugten Ausführungsform der Erfindung ist eine Fixiereinrichtung vorgesehen, zur Fixierung der beiden Versteifungsabschnitte in einer geforderten Relativposition. Die Fixiereinrichtung weist in vorteilhafter Weise eine Fixiermechanik insbesondere eine Rastmechanik auf. Vorzugsweise sind mehrere vorgegebene Rastpositionen auswählbar. Alternativ hierzu - oder auch in Kombination mit dieser Maßnahme ist es auch möglich, die Fixiereinrichtung unter Anwendung von Mitteln zur Fixierung durch Klebung, Schweissung oder chemische Reaktion auszubilden.

Eine unter fertigungstechnischen Gesichtspunkten besonders vorteilhafte Ausführungsform der Erfindung ist dadurch gegeben, daß die beiden Versteifungsabschnitte integral ausgebildet sind. Hierbei ist es möglich, die beiden Versteifungsabschnitte beispielsweise aus einem thermoplastischen Kunststoffmaterial in einem lediglich zweiteiligen Formwerkzeug zu bilden. Die beiden Versteifungsabschnitte können in einer unter entformungstechnischen Gesichtspunkten vorteilhaften Ausrichtung gespritzt werden.

Das Versteifungselement ist vorzugsweise rahmen- skelett- oder zumindest im Bereich der Dichtlippeneinrichtung glockenartig ausgebildet. Im Falle einer skelett- oder rahmenartigen Ausgestaltung weisen die einzelnen Stegabschnitte vorzugsweise im wesentlichen rechteckförmige Profilquerschnitte auf. Hierbei wird eine hohe Steifigkeit unter einem geringen Eigengewicht des Versteifungselementes erreicht.

Der erste Versteifungs- oder Rahmenabschhnitt weist vorzugsweise eine im wesentlichen der Maskenauflagezone entsprechende Kontur auf. Der zweite Versteifungs- oder Rahmenabschnitt erstreckt sich vorzugsweise bis in einen, in Applikationsposition der Maske oberhalb der Augenbrauen des Maskenanwenders befindenden Bereich hinein.

Eine insbesondere bei der Verwendung eines elastomeren Maskenbasiskörpers vorteilhafte Ausführungsform der Erfindung ist dadurch gegeben, daß das Versteifungselement mit Koppelungsabschnitten versehen ist, zur Ankoppelung einer Kopfbandeinrichtung. Hierbei wird es möglich, die Maskenhaltekräfte ohne unzulässige Deformation der Maske einzuleiten.

Das Versteifungselement ist vorzugsweise aus einem Kunststoffmaterial gebildet. Alternativ hierzu - oder auch in Kombination damit - ist es möglich, das Versteifungselement aus einem Metallwerkstoff insbesondere einem biegeverformbaren Draht- oder Profilmaterial zu fertigen. Es ist auch möglich, das Versteifungselement aus einem zumindest lokal thermoverformbaren Material insbesondere einem thermoplastischen Material mit zugsteifer Einlage zu bilden.

Insbesondere ist es möglich, in dem Bereich der biegeneutralen Zone des Profilmateriales eine Drahteinlage vorzusehen.

Das Profilmaterial weist vorzugsweise wenigstens eine zugsteife Ader, beispielsweise aus einem Drahtwerkstoff auf. Es ist möglich, zumindest lokal eine Verformung des Profilmateriales beispielsweise durch Erwärmung, zu ermöglichen, so daß eine weitere Anpassbarkeit des Versteifungs- oder Rahmenelementes an die Gesichtstektur ermöglicht ist.

Eine besonders vorteilhafte Anpassbarkeit der Atemmaske an die Gesichtstektur des Maskenanwenders wird gemäß einer besonders bevorzugten Ausführungsform der Erfindung dadurch erreicht, daß die Atemmaske einen aus einem elastomeren Material gebildeten Maskenbasiskörper aufweist. Hierbei wird es möglich, durch willkürliche Verformung des ersten Versteifungsabschnittes den Verlauf der Dichtlippenauflagezone sowie die Fläachenpressungsverteilung in dieser Zone zu beeinflussen.

In vorteilhafter Weise umfaßt die Stirnauflageeinrichtung ein Auflageelement das aus einem elastomeren Material gebildet ist.

Eine unter hygienischen Gesichtspunkten besonders vorteilhafte Ausführungsform der Erfindung ist dadurch gegeben, daß die Dichtlippeneinrichtung integral mit dem Maskenbasiskörper ausgebildet ist. Diese integrale Ausgestaltung kann erreicht werden durch gemeinsame Bildung in einem Formwerkzeug oder auch durch Verklebung der Dichtlippeneinrichtung mit dem Maskenbasiskörper vorzugsweise unter Einschluß der Versteifungseinrichtung. Es ist auch möglich, den Maskenbasiskörper und die Dichtlippeneinrichtung sowie vorzugsweise auch die Polsterorgane der Stirnauflageeinrichtung im Rahmen eines Vulkanisationsvorganges auszubilden.

Gemäß einem besonderen Aspekt der vorliegenden Erfindung sind die Polsterorgane der Stirnauflageeinrichtung vorzugsweise integral mit der Dichtlippeneinrichtung und/oder dem Maskenbasiskörper ausgebildet. Im Falle einer mehrteiligen Ausgestaltung der Maske ist es möglich, die Dichtlippeneinrichtung über das Versteifungselement mit dem Maskenbasiskörper zu koppeln. Der Verbindungsbereich zwischen dem Maskenbasiskörper und der Stirnauflageeinrichtung kann als elastomere Gelenkstruktur wirksam sein.

Das Versteifungselement ist gemäß einer besonders bevorzugten Ausführungsform der Erfindung lösbar mit der Dichtlippeneinrichtung und/oder dem Maskenbasiskörper gekoppelt. Dadurch wird es auf vorteilhafte Weise möglich, das Versteifungselement mehrfach zu verwenden. Das Versteifungselement ist hierbei vorzugsweise über eine Rast- oder Eingriffsprofilstruktur mit dem Maskenbasiskörper gekoppelt.

Das Versteifungselement ist vorzugsweise mehrteilig ausgebildet. In vorteilhafter Weise ist der Maskenbasiskörper aus einem transparenten oder transluzenten Elastomermaterial gebildet. Eine im Hinblick auf einen hohen Tragekomfort besonders vorteilhafte Ausführungsform der Erfindung ist dadurch gegeben, daß die Dichtlippeneinrichtung eine samtartig mattierte Oberfläche aufweist. Durch die Verwirklichung von sog. Lotusblütenstrukturen wird hierbei auch eine unter hygienischen Gesichtspunkten verbesserte Reinigungsmöglichkeit erreicht.

Die Gelenkfixierung kann vorzugsweise auch reversibel, beispielsweise durch Heißkleber oder chemisch lösbare Klebemittel erfolgen. Es ist auch möglich, speziell im Bereich der Gelenkstelle thermoverformbare Strukturen vorzusehen, die wiederholt plastisch verformbar sind und beispielsweise unter Wärmezufuhr eine erneute Justierung der Relativpositionen der beiden Versteifungs- oder Rahmenabschnitte zulassen.

Es ist auch möglich, in dem Versteifungselement, bzw. in dessen Rahmenstruktur mehrere Gelenk oder Justierzonen auszubilden, so daß beispielsweise auch Einstellmöglichkeiten zur Anpassung an die individuelle Stirnwölbung, die Nasenwurzelbreite und die Oberlippentektur möglich sind.

Insbesondere bei der Ausgestaltung des Versteifungselementes als lokal verformbare Struktur kann bei geringem Bauraum eine ausreichende Festigkeit erreicht werden, indem das Versteifungselement aus einem Verbundwerkstoff gebildet ist. Als Verbundwerkstoff eignet sich insbesondere ein Draht-/Thermoplast-verbundmaterial.

Die Einstellbarkeit der wenigstens zwei Versteifungs- oder Rahmenabschnitte zueinander kann erfindungsgemäß auch dadurch erreicht werden, daß die beiden Versteifungs- oder Rahmenabschnitte in unterschiedlichen Koppelungspositionen zusammenfügbar sind, beispielsweise durch entsprechend permutierbar koppelbare Fügestellen oder auswählbare Fügeglieder.

Die folgenden Punkte werden diskutiert:
1. Atemmaske zur Zufuhr eines Atemgases zu einem Maskenanwender mit: einer Dichtlippeneinrichtung zur Abdichtung einer Maskenauflagezone, einem Maskenbasiskörper zur Bildung eines Maskeninnenraumes und einer Anschlußeinrichtung zum Anschluß wenigstens einer Atemgasleitung wobei der Maskenbasiskörper und/oder die Anschlußeinrichtung mit wenigstens einer aus einem elastomeren Material gebildeten Entkoppelungsstruktur versehen ist, die eine Relativbewegung zwischen einem maskenbasiskörperseitigen Umfangsfußbereich der Dichtlippeneinrichtung und der Atemgasleitung In einem Kippwinkelbereich von wenigstens 5° zuläßt.
2. Atemmaske nach Punkt 1, dadurch gekennzeichnet, daß die Entkoppelungsstruktur durch eine Falten- oder Rollbalgstruktur gebildet ist.
3. Atemmaske nach Punkt 2, dadurch gekennzeichnet, daß die Falten- oder Rollbalgstruktur an der Anschlußeinrichtung ausgebildet ist.
4. Atemmaske nach wenigstens einem der Punkte 1 bis 3, dadurch gekennzeichnet, daß die Entkoppelungsstruktur in dem Maskenbasiskörper ausgebildet ist.
5. Atemmaske nach wenigstens einem der Punkte 1 bis 4, dadurch gekennzeichnet, daß im Bereich der Entkoppelungsstruktur wenigstens eine Kanaleinrichtung ausgebildet ist, zur Schaffung einer Verbindung zwischen dem Maskeninnenbereich und der Umgebung.
6. Atemmaske nach wenigstens einem der Punkte 1 bis 5, dadurch gekennzeichnet, daß die Kanaleinrichtung durch wenigstens eine Durchgangsöffnung gebildet ist.
7. Atemmaske nach wenigstens einem der Punkte 1 bis 6, dadurch gekennzeichnet, daß die Durchgangsöffnung einen Querschnitt aufweist der auf eine vorgegebene Druck/Volumenstromcharakteristik abgestimmt ist.
8. Atemmaske nach wenigstens einem der Punkte 1 bis 7, dadurch gekennzeichnet, daß die Kanaleinrichtung sich hinsichtlich ihres Querschnittes in Strömungsrichtung kontinuierlich oder stufenartig verjüngt.
9. Atemmaske nach wenigstens einem der Punkte 1 bis 8, dadurch gekennzeichnet, daß in einem Austrittsmündungsbereich der Kanaleinrichtung eine scharfe Mündungskante ausgebildet ist.
10. Atemmaske nach wenigstens einem der Punkte 1 bis 9, dadurch gekennzeichnet, **daß** in der Kanaleinrichtung ein Abschnitt engsten Querschnitts definiert ist, und daß die Länge des Abschnitts engsten Querschnitts kleiner ist als 2mm.
11. Atemmaske nach Punkt 10, dadurch gekennzeichnet, daß der engste Querschnitt der Kanaleinrichtung in einem Membranelement ausgebildet ist.
12. Atemmaske nach Punkt 11, dadurch gekennzeichnet, daß die Fiäche des Membranelementes wenigstens 30% größer ist als der Querschnitt der darin gebildeten Drosseldurchgangsöffnung.
13. Atemmaske nach wenigstens einem der Punkte 1 bis 12, dadurch gekennzeichnet, daß die Entkoppelungsstruktur sich in dem Maskenbasiskörper in Umfangsrichtung der Dichtlippeneinrichtung entlang eines Übergangsbereiches zwischen einem Fußbereich der Dichtlippeneinrichtung erstreckt und eine Umfangsfalte oder eine Rollbalgstruktur aufweist.
14. Atemmaske zur Zufuhr eines Atemgases zu einer Person mit: einer Dichtlippeneinrichtung zur Abdichtung einer Maskenauflagezone und einer Stirnauflageeinrichtung zur Abstützung der Maske im Stirn- oder Nasenwurzelbereich der Person insbesondere nach wenigstens einem der Punkte 1 bis 13. gekennzeichnet durch ein Versteifungselement, zur Aussteifung der Atemmaske mit einem dem Bereich der Dichtlippeneinrichtung zugeordneten ersten Versteifungsabschnitt und einem der Stirnauflageeinrichtung zugeordneten zweiten Versteifungsabschnitt, wobei die Relativposition der beiden Versteifungsabschnitte zueinander einstellbar veränderbar ist.
15. Atemmaske nach Punkt 14, dadurch gekennzeichnet, daß die beiden Versteifungsabschnitte über eine Geienkeinrichtung miteinander gekoppelt sind.
16. Atemmaske nach Punkte 14 oder 15, dadurch gekennzeichnet, daß eine Fixiereinrichtung vorgesehen ist, zur Fixierung der beiden Versteifungsabschnitte in einer geforderten Relativposition.
17. Atemmaske nach wenigstens einem der Punkte 14 bis 16, dadurch gekennzeichnet, daß die beiden Versteifungsabschnitte integral ausgebildet sind.
18. Atemmaske nach wenigstens einem der Punkte 14 bis 37, dadurch gekennzeichnet, daß die Gelenkeinrichtung durch eine Filmscharniereinrichtunggebildet ist.
19. Atemmaske nach wenigstens einem der Punkte 14 bis 38, dadurch gekennzeichnet, daß das Versteifungselement auf die lokal wirkenden Kräfte abgestimmt skelet- glocken- oder rahmenartig ausgebildet ist.
20. Atemmaske nach wenigstens einem der Punkte 14 bis 14, dadurch gekennzeichnet, daß das Versteifungselement durch ein im wesentlichen rechteckförmiges Rahmenprofil aufweist.
21. Atemmaske nach wenigstens einem der Punkte 14 bis 20, dadurch gekennzeichnet, daß die Fixiereinrichtung eine Fixiermechanik insbesondere eine Rastmechanik aufweist.
22. Atemmaske nach wenigstens einem der Punkte 14 bis 21, dadurch gekennzeichnet, daß die Fixiereinrichtung Mittel zur Fixierung durch Klebung, Schweissung oder chemische Reaktion aufweist.
23. Atemmaske nach wenigstens einem der Punkte 14 bis 22, dadurch gekennzeichnet, daß das der erste Versteifungs- oder Rahmenabschnitt eine im wesentlichen der Maskenauflagezone entsprechende Kontur aufweist.
24. Atemmaske nach wenigstens einem der Punkte 14 bis 23, dadurch gekennzeichnet, daß der zweite Versteifungsabschnitt sich bis in einen, in Applikationsposition der Maske oberhalb der Augenbrauen des Maskenanwenders befindenden Bereich hinein erstreckt.
25. Atemmaske nach wenigstens einem der Punkte 14 bis 24, dadurch gekennzeichnet, daß das Versteifungselement mit Koppelungsabschnitten versehen ist, zur Ankoppelung einer Kopfbandeinrichtung.
26. Atemmaske nach wenigstens einem der Punkte 14 bis 25, dadurch gekennzeichnet, daß das Versteifungselement aus einem Kunststoffmaterial und/oder einem vorzugsweise thermoverformbaren Material insbesondere Draht-Kunststoffverbundmaterial gebildet ist.
27. Atemmaske nach wenigstens einem der Punkte 14 bis 26, dadurch gekennzeichnet, daß die Atemmaske einen aus einem elastomeren Material gebildeten Maskenbasiskörper aufweist.
28. Atemmaske nach wenigstens einem der Punkte 14 bis 27, dadurch gekennzeichnet, daß die Dichtlippeneinrichtung integral mit dem Maskenbasiskörper ausgebildet ist.
29. Atemmaske nach wenigstens einem der Punkte 14 bis 28, dadurch gekennzeichnet, daß die Dichtlippeneinrichtung über das Versteifungselement mit dem Maskenbasiskörper gekoppelt ist.
30. Atemmaske nach wenigstens einem der Punkte 14 bis 29, dadurch gekennzeichnet, daß die Stirnauflageeinrichtung ein Auflageelement aufweist das aus einem elastomeren Material gebildet ist.
31. Atemmaske nach wenigstens einem der Punkte 14 bis 30, dadurch gekennzeichnet, daß das Auflageelement integral mit der Dichtlippeneinrichtung und/oder dem Maskenbasiskörper ausgebildet ist.
32. Atemmaske nach wenigstens einem der Punkte 14 bis 31, dadurch gekennzeichnet, daß das Versteifungselement lösbar mit der Dichtlippeneinrichtung und/oder dem Maskenbasiskörper gekoppelt ist.
33. Atemmaske nach wenigstens einem der Punkte 14 bis 32, dadurch gekennzeichnet, daß das Versteifungselement über eine Rast- oder Eingriffsprofilstruktur mit dem Maskenbasiskörper gekoppelt ist.
34. Atemmaske nach wenigstens einem der Punkte 14 bis 33, dadurch gekennzeichnet, daß das Versteifungselement mehrteilig ausgebildet ist.
35. Atemmaske nach wenigstens einem der Punkte 14 bis 34, dadurch gekennzeichnet, daß der Maskenbasiskörper aus einem transparenten oder transluzenten Elastomermaterial gebildet ist.
36. Atemmaske nach wenigstens einem der Punkte 14 bis 35, dadurch gekennzeichnet, daß die Dichtiippeneinrichtung und/oder die elastomere Entkoppelungsstruktur eine samtartig mattierte Oberfläche aufweist.
37. Ableitungseinrichtung zur Ableitung von Atemgas mit einem Anschlußabschnitt zur Ankoppelung einer Atemgasleitung und einer Umfangswandung zur Begrenzung eines Atemgasdurchgangsweges wobei die Umfangswandung zumindest abschnittsweise mit einer elastomeren Gelenkstruktur versehen ist, und im Bereich der Gelenkstruktur wenigstens eine Durchgangsöffnung vorgesehen ist, zur Schaffung einer Verbindung zwischen dem Atemgasdurchgangsweges und der Umgebung.
38. Ableitungseinrichtung nach Punkt 37, dadurch gekennzeichnet, daß die Durchgangsöffnung derart angeordnet ist, daß eine Abströmung des Atemgases in einen über den Augenbrauen liegenden Bereich erfolgt.
39. Ableitungseinrichtung nach Punkt 37 oder 38, dadurch gekennzeichnet, daß mehrere Durchgangsöffnungen vorgesehen sind.
40. Ableitungseinrichtung nach wenigstens einem der Punkte 37 bis 39, dadurch gekennzeichnet, daß die Durchgangsöffnungen in einer sich im wesentlichen in radialer Richtung erstreckenden Wandung eines Faltenkranzes ausgebildet sind.
41. Ableitungseinrichtung nach wenigstens einem der Punkte 37 bis 40, dadurch gekennzeichnet, daß die Durchgangsöffnungen jeweils eine Querschnittsfläche im Bereich von 0,8 bis 4 mm² aufweisen, und daß die Durchgangsöffnungen derart ausgebildet sind, daß die Abströmrichtung im wesentlichen normal zur umgebenden Wandung des Falteneinzuges erfolgt.
42. Ableitungseinrichtung nach wenigstens einem der Punkte 37 bis 41, dadurch gekennzeichnet, daß zwischen benachbarten Durchgangsöffnungen ein Stegabschnitt ausgebildet ist, zur Knick- oder Biegebegrenzung.
43. Ableitungseinrichtung nach wenigstens einem der Punkte 37 bis 42, dadurch gekennzeichnet, daß mehrere Umfangsfalteneinzüge vorgesehen sind, und daß die Durchgangsöffnungen in den radialen Wandungen zweier abfoigender Umfangsfalteneinzüge ausgebildet sind.
44. Abieitungseinrichtung nach wenigstens einem der Punkte 37 bis 42, dadurch gekennzeichnet, daß die Durchgriffsöffnungen derart ausgerichtet angeordnet sind, daß die Abströmung entlang der Außenflächen eines Atemgasschlauches erfolgt.

Weitere Einzelheiten ergeben sich aus der nachfolgenden Beschreibung in Verbindung mit der Zeichnung. Es zeigen:
- **Fig. 1**: eine perspektivische Ansicht einer bevorzugten Ausführungsform einer Atemmaske mit einem elastomeren Maskenbasiskörper und integraler Stirnauflageeinrichtung, wobei ein Versteifungselement vorgesehen ist über welches die Position der Stirnaufiageeinrichtung relativ zu einer mit dieser integralen Dichtiippeneinrichtung einstellbar veränderbar ist;
- **Fig. 2**: eine perspektivische Ansicht des rahmenartig ausgebildeten Versteifungselementes wie es bei der Ausführungsform gem Fig. vorgesehen ist;
- **Fig. 3**: eine weitere perspektivische Ansicht des genannten Versteifungselementes hier mit Blick auf ein in mehreren Fixierpositionen verrastbares Fixierorgan.
- **Fig. 4**: eine vereinfachte perspektivische Ansicht einer weiteren Ausführungsform einer Atemmaske mit einer im Bereich einer Atemgasleitungseinrichtung ausgebildeten Eiastomer-Entkoppeiungsstruktur:
- **Fig. 5**: eine perspektivische Ansicht einer dritten Ausführungsform einer Atemmaske mit einem aus einem Elastomermaterial gebildeten Maskenbasiskörper und einer an diesem ausgebildeten, durch Falteneinzüge gebildeten Entkoppelungsstruktur;
- **Fig. 6**: eine perspektivische Ansicht einer vierten Ausführungsform einer Atemmaske, ebenfalls mit einem aus einem Elastomermaterial gebildeten Maskenbasiskörper, jedoch mit einer nach dem Rollbalg-Prinzip ausgebildeten Entkoppelungsstruktur;
- **Fig. 7**: eine Seitenansicht einer fünften Ausführungsform einer Atemmaske, ebenfalls mit einem aus einem Elastomermaterial gebildeten Maskenbasiskörper sowie einer daran angeformten Anschlußeinrichtung für eine Atemgasleitung, wobei sowohl zwischen der Anschiußeinrichtung und dem Maskenbasiskörper, als auch im Bereich der Dichtlippeneinrichtung jeweils eine Entkoppelungsstruktur ausgebildet ist;
- **Fig. 8**: eine bevorzugte Ausführungsform eines Anschlußabschnittes für eine Atemgasleitung, hier mit integrierten Gasableitungsäffnungen;
- **Fig. 9**: eine perspektivische Ansicht einer weiteren Ausführungsform einer Anschlußeinrichtung für eine Atemgasleitung, hier mit mehreren umflaufenden Faltenbälgen sowie eingeformten Ableitungsöffnungen;
- **Fig. 10**: eine vereinfachte Schnittansicht zur Erläuterung einer weiteren Ausführungsform einer Kanaleinrichtung zur Ableitung von Atemgas;
- **Fig. 11a, Fig. 11b**: Prinzipskizzen zur Erläuterung des Aufbaus einer als Kraft- und/oder Druckanzeigeeinrichtung geeigneten verformbaren Struktur;
- **Fig. 12**: eine Detailskizze zur Erläuterung einer bevorzugten Anordnung von Versteifungsstegen zur Verschlußverhinderung der Durchgangsöffnungen.

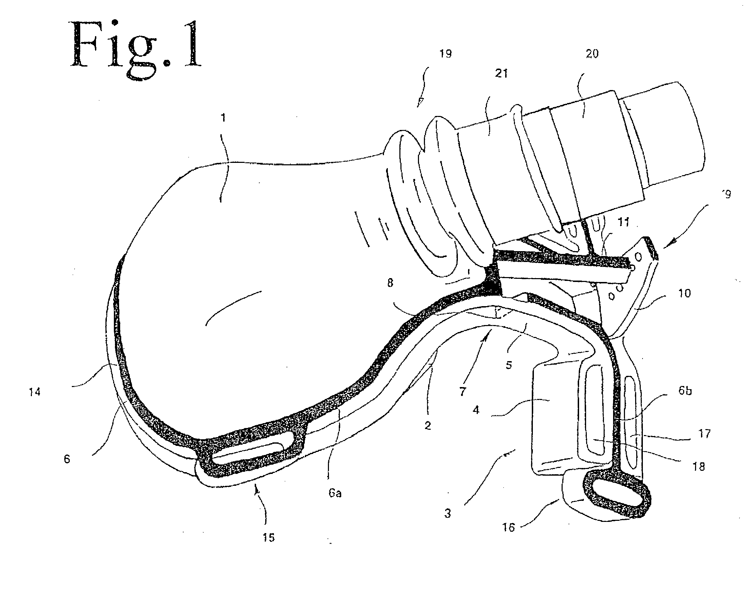

Die Darstellung gem. Fig. 1 zeigt eine Atemmaske, wie sie insbesondere für die Durchführung einer CPAP-Therapie Anwendung finden kann. Die Atemmaske umfaßt einen Maskenbasiskörper 1, der bei der hier dargestellten Ausführungsform aus einem volltransparenten, elastomeren Silikonmaterial gebildet ist. Der Maskenbasiskörper 1 definiert einen zur Aufnahme der Nase eines Maskenanwenders ausreichend bemessenen Maskeninnenraum. Die Abdichtung des Maskeninnenraumes zur Gesichtsfläche des Maskenanwenders hin erfolgt über eine Dichtlippeneinrichtung 2, die in dieser Ansicht nahezu vollständig verdeckt ist. Die Dichtlippeneinrichtung 2 umfaßt eine aus einem Elastomermaterial gebildete Dichtlippe, die sich unter elastischer Verformung auf die Gesichtsfläche des Maskenanwenders legt und eine Nasenaufnahmeöffnung definiert, durch welche zumindest der Nasenspitzenbereich des Maskenanwenders in den durch die Maskenbasiskörper 1 definierten Maskeninnenraum vordringen kann.

Die Atemmaske ist mit einer Stirnauflageeinrichtung 3 versehen, die hier eine Stirnpolstereinrichtung 4 umfaßt. Die Stirnpolstereinrichtung 4 ist bei der hier dargestellten Ausführungsform ebenfalls aus einem Elastomermaterial und zudem integral mit der Dichtlippeneinrichtung 2 sowie integral mit dem Maskenbasiskörper 1 ausgebildet. Diese Integralausbildung von Maskenbasiskörper 1, Dichtlippeneinrichtung 2 und Stirnpolstereinrichtung 4 wird unter Ausbildung eines Verbindungsstegabschnittes 5 erreicht, welcher die Stirnpolstereinrichtung 4 mit dem Maskenbasiskörper 1 positionsveränderbar koppelt. Im Umfangsbereich des Maskenkörpers 1 ist ein Versteifungselement 6 vorgesehen, das hier als rahmenartige Struktur ausgebildet ist. Das Versteifungselement 6 umfaßt einen der Umfangskontur der Dichtlippeneinrichtung 2 folgenden ersten Versteifungsabschnitt 6a sowie einen sich in die Stirnauflageeinrichtung 3 hineinerstreckenden zweiten Versteifungsabschnitt 6b. Die beiden Versteifungsabschnitte 6a, 6b sind zueinander bewegbar gekoppelt. Die Koppelung der beiden Versteifungsabschnitte 6a, 6b erfolgt bei der hier dargestellten Ausführungsform über eine Gelenkeinrichtung 7, die hier durch einen Filmscharnierabschnitt 8 gebildet ist. Die durch Schwenken der beiden Versteifungsabschnitte 6a, 6b zueinander eingestellte Relativposition der Stirnpolstereinrichtung 4 relativ zu dem Maskenbasiskörper. 1 bzw. zu der Dichtlippeneinrichtung 2 kann über eine Fixiereinrichtung 9 dauerhaft festgelegt werden.

Die Fixiereinrichtung 9 umfaßt hier ein Fixierelement 10, das mit einem Halteelement 11 in unterschiedlichen Fixierpositionen in Eingriff bringbar ist.

Bei der hier gezeigten Ausführungsform ist das Halteelement 11 weitgehend biegesteif mit dem ersten Verbindungsabschnitt 6a verbunden. Das Fixierelement 10 ist schwenkbewegbar mit dem zweiten Versteifungsabschnitt 6b verbunden. Die Koppelung des Fixierelementes 10 und des Halteelements 11 in ausgewählten Koppelungspositionen erfolgt hier über Steckbohrungen 12, die in dem Fixierelement 10 ausgebildet sind. Diese Steckbohrungen 12 sind mit einem hier nicht sichtbaren Fixierzapfen in Eingriff bringbar. Der Fixierzapfen ist an einer dem Fixierelement 10 zugewandten Stirnfläche des Halteelementes 11 ausgebildet. Alternativ zu diesem hier gezeigten Mechanismus ist es auch möglich, abweichende Mechanismen zur Festlegung der Relativposition des ersten Versteifungsabschnittes 6a gegenüber dem zweiten Versteifungsabschnitt 6b heranzuziehen.

Die Versteifungseinrichtung 6 ist mit dem Maskenbasiskörper 1 derart gekoppelt, daß der Maskenbasiskörper 1 und mit diesem auch die Dichtlippeneinrichtung 2 eine durch das Versteifungselement 6 mitbestimmte Gestalt aufweisen. Bei der hier dargestellten Ausführungsform erfolgt die Koppelung des Versteifungsefementes 6 mit dem Maskenbasiskörper 1 über eine Umfangsnut 14, in welche das rahmenartig ausgebildete Versteifungselement 6 eingesetzt ist. Zur besseren Koppelung des Versteifungselementes 6 mit dem Maskenbasiskörper 1, bzw. mit der Dichtlippeneinrichtung 2 ist die Umfangsnut im Bereich ihrer Nutinnenflächen komplementär zu dem Versteifungselement 6 profiliert.

Das Versteifungselement 6 ist mit einer Koppelungseinrichtung 15 versehen, über welche ein Bandelement einer unteren Kopfbandanordnung mit der Atemmaske koppelbar ist. Bei der hier dargestellten Ausführungsform ist die Koppelungseinrichtung 15 als bügelartige Lasche ausgebildet, die integral mit dem Versteifungselement 6 gefertigt ist. Alternativ hierzu ist es auch möglich, konstruktiv abweichend aufgebaute Koppelungseinrichtungen, beispielsweise Rast- oder Schnappeinrichtungen zur Koppelung des Kopfbandes mit dem Versteifungselement 6 heranzuziehen. Durch die unmittelbare Einleitung der Zugkräfte des Kopfbandes in das Versteifungselement 6 wird eine unzulässige Deformation des Maskenbasiskörpers 1 sowie der Dichtlippeneinrichtung 2 vermieden.

Im Bereich der Stirnauflageeinrichtung 3 ist ebenfalls eine Koppelungseinrichtung 16 vorgesehen, die bei der hier dargestellten Ausführungsform in ihrem Aufbau im wesentlichen der im Bereich des Maskenbasiskörpers 1 vorgesehenen Koppelungseinrichtung 15 entspricht.

Das im Bereich der Stirnauflageeinrichtung 3 vorgesehene Versteifungselement 6 bzw. dessen zweiter Versteifungs- oder Rahmenabschnitt 6b ist mit der Stirnpolstereinrichtung 4 verbunden. Bei der hier gezeigten Ausführungsform erfolgt die Koppelung der Stirnpolstereinrichtung 4 mit dem zweiten Versteifungsabschnitt 6b, ähnlich wie die Koppelung des ersten Versteifungsabschnittes 6a mit dem Maskenbasiskörper 1 dadurch, daß der zweite Versteifungsabschnitt 6b in eine, in der Stirnpolstereinrichtung 4 ausgebildete Nut eingesteckt ist.

Die Stirnpolstereinrichtung 4 ist aus einem elastomeren Material gebildet und weist mehrere Taschenabschnitte 17, 18 auf. Durch die Taschenabschnitte 17, 18 können die Polstereigenschaften der Stirnpolstereinrichtung 4 definiert beeinflußt werden.

An dem Maskenbasiskörper 1 ist eine Balgstruktur 19 vorgesehen, über welche ein Atemschlauchanschlußadapter 20 schwenkbewegbar mit dem Maskenbasiskörper 1 gekoppelt ist.

Die Balgstruktur 19 ist bei der hier dargestellten Ausführungsform ebenfalls integral mit dem Maskenbasiskörper 1 ausgebildet, wodurch in unter hygienischen Gesichtspunkten vorteilhafter Weise etwaigen Spaltbildungen vorgebeugt ist. An die Balgstruktur 19 schließt sich ein Schlauchzapfenabschnitt 21 an, welcher hinsichtlich seines Innendurchmessers derart bemessen ist, daß der Atemschlauchanschlußadapter 20 in diesen festsitzend einsteckbar ist. Anstelle des Atemschlauchanschlußadapters 20 ist es möglich, hier einen CO₂-Auswaschadapter vorzusehen, wie er in der auf die Anmelderin zurückgehenden deutschen Patentschrift 198 40 760.2 beschrieben ist.

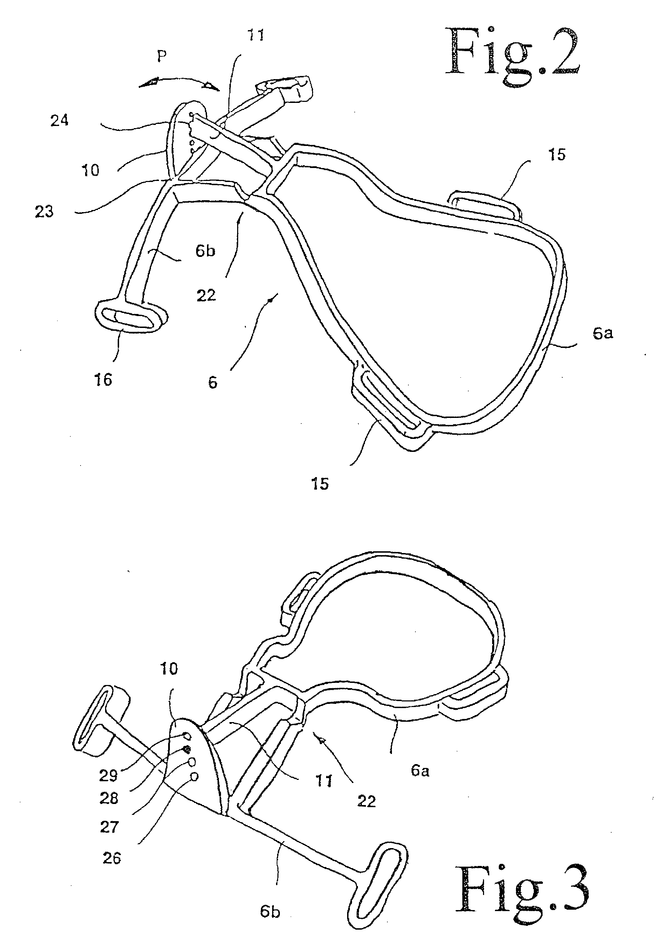

In Fig. 2 ist das bei der Atemmaske gem. Fig. 1 vorgesehene Versteifungselement 6 als Einzelteil dargestellt. Sowohl der dem Maskenbasiskörper 1 der Atemmaske zugeordnete erste Versteifungsabschnitt 6a als auch der der Stirnauflageeinrichtung 3 (Fig. 1) zugeordnete zweite Versteifungsabschnitt 6b sind hier durch stegartige Elemente gebildet, die einen im wesentlichen polygonalen, insbesondere rechteckförmigen Querschnitt aufweisen. Integral mit diesen stegförmigen Elementen sind die bereits in Verbindung mit Fig. 1 angesprochenen Koppelungseinrichtungen 15, 16 ausgebildet. Deutlich erkennbar ist in dieser Darstellung die hier als Filmscharnier 22 ausgebildete Koppelungsstelle zur Koppelung der beiden Versteifungsabschnitte 6a, 6b. Das Fixierelement 10 ist ebenfalls über eine Filmscharnierstelle 23 derart gelagert, daß dieses, wie durch den Pfeil P angedeutet, in einem ausreichenden Winkelbereich schwenkbar ist. Durch entsprechendes Schwenken des Fixierelementes 10 wird erreicht, daß dieses einen Fixierzapfen 24, der im Endbereich des Halteelementes 11 ausgebildet ist, freigibt. Solange das Fixierelement 10 nicht mit dem Halteelement 11 gekoppelt ist, ist es möglich, den zweiten Versteifungsabschnitt 6b relativ zu dem ersten Versteifungsabschnitt 6a in eine gewünschte Position zu schwenken. Durch in Eingriffbringen der an dem Fixierelement 10 vorgesehenen Eingriffstruktur mit einer entsprechend komplementären Eingriffstruktur des Halteelementes 11 wird es möglich, die beiden Versteifungsabschnitte 6a, 6b in der gewünschten Relativposition zueinander zu fixieren. Das Fixierelement 10, das Halteelement 11 und der sich zwischen dem Filmscharnier 22 zum Filmscharnier 23 hin erstreckende Abschnitt des zweiten Versteifungsabschnittes 6b bilden bei der hier dargestellten Ausführungsform ein Dreiecksfachwerk, durch welches die Relativposition des zweiten Versteifungsabschnittes 6b relativ zu dem ersten Versteifungsabschnitt 6a einstellbar festlegbar ist. Alternativ zu diesem unter Fertigungstechnischen Gesichtspunkten besonders vorteilhaft herstellbaren Koppelungsstruktur ist es auch möglich, konstruktiv abweichende Koppelungsstrukturen zur Koppelung der beiden Versteifungsabschnitte 6a, 6b heranzuziehen.

Der erste Versteifungsabschnitt 6a weist bei der hier dargestellten Ausführungsform eine im wesentlichen sattelförmige Außenkontur auf. In einer bzgl. der Maskenauflagefläche senkrechten Richtung ist der erste Versteifungsabschnitt 6a derart im Nasenrückenbereich nach oben gezogen, daß dieser einen vorbestimmten Minimalabstand zur Gesichtsfläche des Maskenanwenders nicht unterschreitet.

In Fig. 3 ist der Maskenrahmen nach Fig. 2 aus einer anderen Perspektive dargestellt. Das Fixierelement 10 weist hier vier Rastbohrungen 26, 27, 28 und 29 auf. Das Fixierelement 10 ist mit dem Halteelement 11 derart gekoppeit, daß die Rastbohrung 28 mit dem Fixierzapfen 24 des Halteelementes 11 in Eingriff gelangt. Bei Koppelung des Fixierelementes 10 mit dem Halteelement 11 unter Nutzung der Rastbohrung 29 wird der zweite Versteifungsabschnitt 6b derart zum Stirnbereich des Maskenanwenders hingeschwenkt, daß der den Nasenrücken überquerende Bereich des ersten Versteifungsabschnittes 6a am weitesten abgehoben ist. Durch Wahl der Rastbohrung 28 wird eine Konfiguration gewählt, bei welcher der den Nasenrücken überquerende Bereich des ersten Versteifungsabschnittes 6a dem Nasenrücken des Patienten bereits angenähert ist. Durch die Rastbohrungen 27 und 26 werden jeweils noch flachere Konfigurationen erreicht.

Es ist möglich, im Bereich der hier als Filmscharnier 22 ausgebildeten Koppelungsstelle zwischen den beiden Versteifungsabschnitten ein Klebstoffmaterial einzubringen, wodurch die Relativposition der beiden Versteifungsabschnitte 6a, 6b zueinander dauerhaft festgelegt ist. Das Fixierelement 10 und ggf. auch das Halteelement 11 können nach Aushärten des Klebstoffmateriales entfernt werden. Die Einstellung der Atemmaske erfolgt in vorteilhafter Weise unter Verwendung der vollständig zusammenmontierten Atemmaske. Es ist auch möglich, das Versteifungselement in demontiertem Zustand an die Gesichtstektur des Maskenanwenders anzupassen und erst anschließend mit dem Maskenbasiskörper zu koppeln. In vorteilhafter Weise sind weitere Einstelimöglichkeiten vorgesehen, durch welche beispielsweise die Lage der Stirnpolstereinrichtung in Höhen- und/oder in Breitenrichtung einstellbar ist. Alternativ zu der Ausbildung des Koppelungsbereiches zwischen den beiden Versteifungsabschnitten als Gelenkstelle ist es auch möglich, hier Koppelungsmöglichkeiten vorzusehen, durch weiche beispielsweise unter Zufuhr von Wärme eine Einstellmöglichkeit der beiden Versteifungsabschnitte 6a, 6b zueinander gegeben ist.

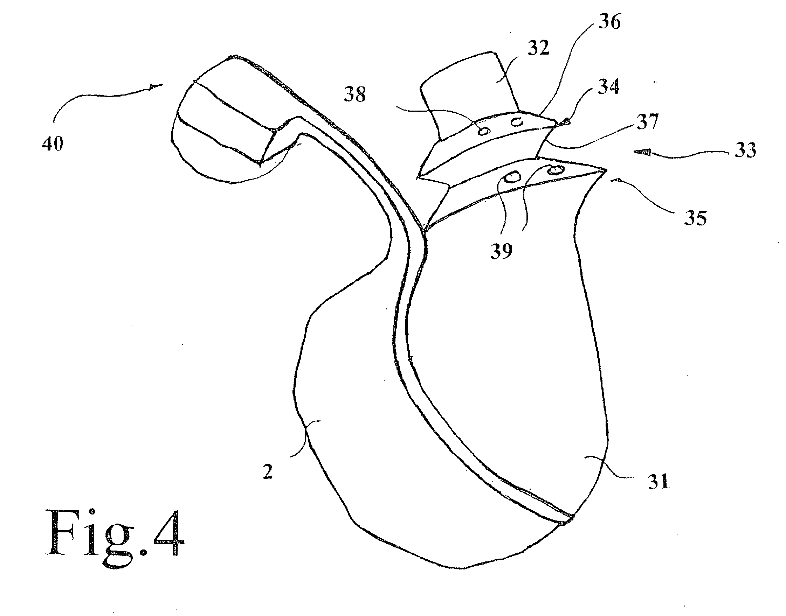

Die in Fig. 4 dargestellte Atemmaske umfaßt eine integral mit dem Maskenbasiskörper 31 ausgebildete Anschlußeinrichtung 32 für eine Atemgasleitung (nicht dargestellt). In einem Übergangsbereich zwischen der Anschlußeinrichtung 32 und dem Maskenbasiskörper 31 ist eine Entkoppelungsstruktur 33 vorgesehen, die bei der hier dargestellten Ausführungsform durch eine Faltenbalgeinrichtung gebildet ist. Die Faktenbafgeinrichtung umfaßt einen ersten Faltenkranz 34 und einen zweiten Faltenkranz 35. Insbesondere der erste Faltenkranz 34 umfaßt zwei sich in radialer Richtung erstreckende Umfangswandungen 36, 37. Diese beiden Umfangswandungen 36, 37 sind als Kegeimantelflächen ausgebildet und weisen eine im Hinblick auf eine vorbestimmte Systemsteifigkeit ausgewählte Wanddickenverteilung auf. Die Entkoppelungsstruktur ist hier rotationssymmetrisch ausgebildet. Ein besonders großer Atemgasdurchtrittsquerschnitt bei vergleichsweise geringer Beinträchtigung des Gesichtsfeldes wird in vorteilhafter Weise erreicht indem der Atemgasleitungsabschnit im Bereich der Nasenwurzel einen elliptischen oder polygonalen Querschnitt aufweist. Die Entkoppelungsstruktur ist hier rotationssymmetrisch ausgebildet. Alternativ hierzu ist es jedoch auch möglich, die Flanken der Faltenkränze derart auszubilden, daß diese in Umfangsrichtung unterschiedliche Einzugstiefen und ggf. wechselnde Wanddicken aufweisen.

Bei der hier dargestellten Ausführungsform ist in der, in Applikationsposition der Atemmaske dem Stirnbereich eines Maskenanwenders zugewandten Umfangswandung 36 wenigstens eine Atemgasdurchgangsöffnung 38 ausgebildet, über welche eine Abströmung von zumindest teilweise verbrauchtem Atemgas in die Umgebung erfolgen kann. Auch im Bereich des zweiten Faltenkranzes 35 sind mehrere Atemgasdurchgangsöffnungen 39 ausgebildet, durch welche ebenfalls eine über den Stirnbereich des Maskenanwenders hinweg gerichtete Atemgasabströmung erfolgen kann. Die Atemgasdurchgangsöffnungen 38 und 39 sind derart ausgerichtet, daß das hieraus austretende Atemgas nicht unmittelbar mit Wandungsabschnitten der Entkoppelungsstruktur oder auch der Anschlußeinrichtung kollidiert. Vorzugsweise sind die Atemgasdurchgangsöffnungen 38, 39 derart angeordnet, daß die austretende Luft nicht auf den Stirnbereich des Maskenanwenders auftreffen kann.

Es ist möglich, die Entkoppelungsstruktur so auszubilden, daß eine Anschlagcharakteristik erreicht wird, so daß eine Entkoppelung nur inerhalb eines bestimmten Bewegungsbereiches zugelasen wird

Bei der hier dargestellten Atemmaske ist integral mit dem Maskenbasiskörper 31 eine Stirnauflageeinrichtung 40 ausgebildet. Die Dichtlippeneinrichtung 2 zur Abdichtung einer Gesichtsauflagezone ist bei der hier dargestellten Ausführungsform ebenfalls integral mit dem Maskenbasiskörper 31, bzw. integral mit der Stirnauflageeinrichtung 40 ausgebildet. Die Position der Stirnauflageeinrichtung 40 gegenüber dem Maskenbasiskörper 31 oder auch gegenüber der Dichtlippeneinrichtung 2 ist über eine Einstelieinrichtung veränderbar.

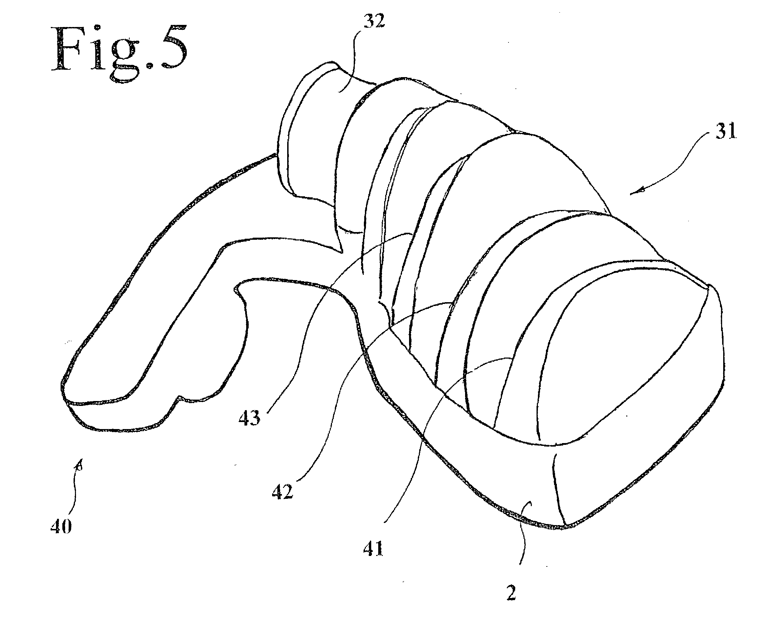

Bei der in Fig. 5 dargestellten Ausführungsform einer Atemmaske ist der Maskenbasiskörper 31 ebenfalls aus einem Elastomermaterial gebildet, jedoch abweichend von der vorangehend beschriebenen Ausführungsform mit einer Vielzahl von Falteneinzügen versehen, durch welche ebenfalls eine weitgehende mechanische Entkoppelung der Anschlußeinrichtung 32 von der Dichtlippeneinrichtung 2 erreicht wird. Auch bei dieser Ausführungsform weist die Atemmaske eine Stirnauflageeinrichtung 40 auf, die ähnlich wie bei der vorangehend beschriebenen Ausführungsform integral mit dem Maskenbasiskörper 31 ausgebildet sein kann.

Die Falteneinzüge 41, 42, 43 sind hier derart ausgerichtet, daß diese in Applikationsposition der Atemmaske den Nasenrückenbereich bogenartig überbrücken. Alternativ zu der hier gezeigten Ausführungsform mit drei vergleichsweise tiefen Falteneinzügen ist es auch möglich, den Maskenbasiskörper 31 mit einer größeren Anzahl entsprechender Falteneinzüge versehen, wobei die elastischen Eigenschaften der einzelnen Faltenzüge vorzugsweise derart abgestimmt sind, daß sich hinsichtlich der Koppelung der Anschlußeinrichtung 32 und der Dichtlippeneinrichtung 2 vorgegebene Eigenschaften ergeben.

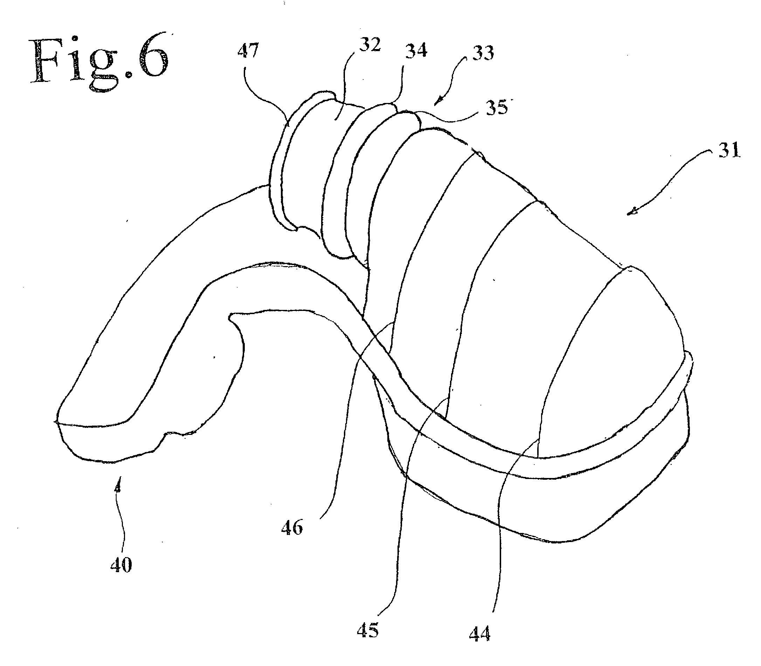

Die in Fig. 6 dargestellte Atemmaske umfaßt ähnlich wie die vorangehend beschriebene Atemmaske eine im Bereich des Maskenbasiskörpers 31 ausgebildete Entkoppelungsstruktur. Bei der hier gezeigten Ausführungsform ist die Entkoppelungsstruktur durch mehrere Rollbalgzonen 44, 45, 46 gebildet. Zusätzlich zu diesen Rollbalgzonen 44, 45, 46 ist im Bereich der Anschlußeinrichtung 32 eine weitere Entkoppelungsstruktur 33 vorgesehen, die ähnlich, wie bei der in Verbindung mit Fig. 4 beschriebenen Ausführungsform zwei Faltenkränze 34, 35 aufweist.

Die Abstützung dieser Atemmaske im Stirnbereich eines Maskenanwenders erfolgt auch hier durch eine Stirnauflageeinrichtung 40 die hier integral mit dem Maskenbasiskörper 31 ausgebildet ist.

Die Anschlußeinrichtung 32 ist hier integral mit dem Maskenbasiskörper 31 ausgebildet und entsprechend ebenfalls aus einem Elastomermaterial gefertigt. Die Anschlußeinrichtung 32 weist einen Umfangswulst 47 auf, über welche eine verbesserte Koppelung mit einer Atemgasleitung erreicht wird.

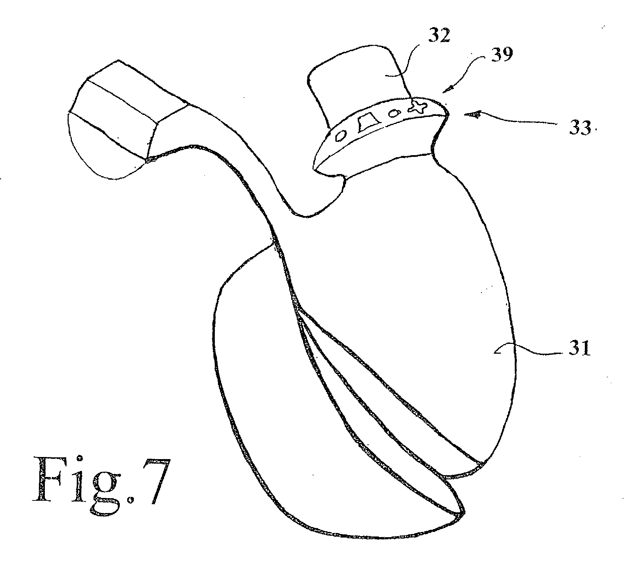

Die in Fig. 7 dargestellte Atemmaske umfaßt einen aus einem Elastomermaterial gefertigten Maskenbasiskörper 31 mit einer ebenfalls aus einem Elastomermaterial gefertigten Anschlußeinrichtung 32. Die Anschlußeinrichtung 32 ist hier integral mit dem Maskenbasiskörper ausgebildet, wobei in einem Übergangsbereich die Anschlußeinrichtung 32 in den Maskenbasiskörper 31 eine, hier durch lediglich einen einzigen Faltenbalg gebildete, Entkoppelungsstruktur 33 vorgesehen ist. Im Bereich der Entkoppelungsstruktur sind mehrere Atemgasdurchgangsöffnungen 39 ausgebildet zur Ableitung von Atemgas aus dem, durch den Maskenbasiskörper 31 definierten Maskeninnenraum. Die Atemgasdurchgangsöffnungen 39 weisen hinsichtlich eines vorbestimmten Abströmverhaltens ausgebildete Kanalquerschnitte auf. Die Atemgasdurchgangsöffnungen 39 können, wie hier beispielhaft dargestellt, nicht nur, wie bei den vorangehend beschriebenen Ausführungen erläutert, runde Querschnitte, sondern auch polygonale, kreuzförmige und andere willkürlich ausgewählte Geometrien aufweisen. Vorzugsweise sind die Atemgasdurchgangsöffnungen jedoch derart ausgebildet, daß die hierdurch erfolgende Luftströmung nicht auf den Stirnbereich eines Maskenanwenders gerichtet ist, sondern insbesondere in Schlauchrichtung insbesondere entlang der dem Maskenanwender abgewandten Seite der Schlauchwandung erfolgt.

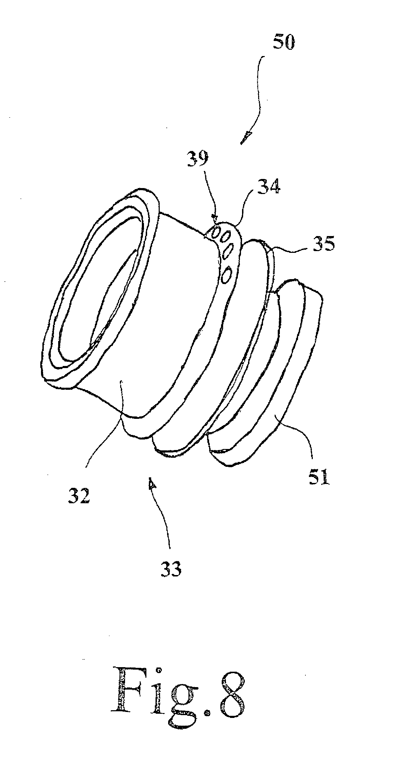

In Fig. 8 ist eine Abteitungseinnchtung 50 zur Ableitung von Atemgas dargestellt. Die Ableitungseinrichtung 50 bildet hier eine Anschlußeinrichtung 32 zum Anschluß einer Atemgasleitung sowie eine Entkoppelungsstruktur 33, die hier mehrere Faltenkränze 34, 35 aufweist. Auf einer der Anschlußeinrichtung 32 abgewandten Seite ist die Ableitungseinrichtung 50 mit einer Befestigungsstruktur 51 versehen, über welche die Ableitungseinrichtung 50 in abdichtender Weise mit einem Maskenbasiskörper einer Atemmaske oder einem weiteren Atemgasleitungsabschnitt koppelbar ist. Im Bereich eines sich über einen Winkel von ca. 120° erstreckenden Umfangsabschnittes des ersten Faltenkranzes 34 sind ähnlich, wie bei den vorangehend beschriebenen Ausführungsformen Atemgasdurchgangsöffnungen 39 ausgebildet, über welche eine Ableitung von Atemgas aus dem durch die Ableitungseinrichtung 50 definierten Innenraum in die Umgebung erfolgen kann.

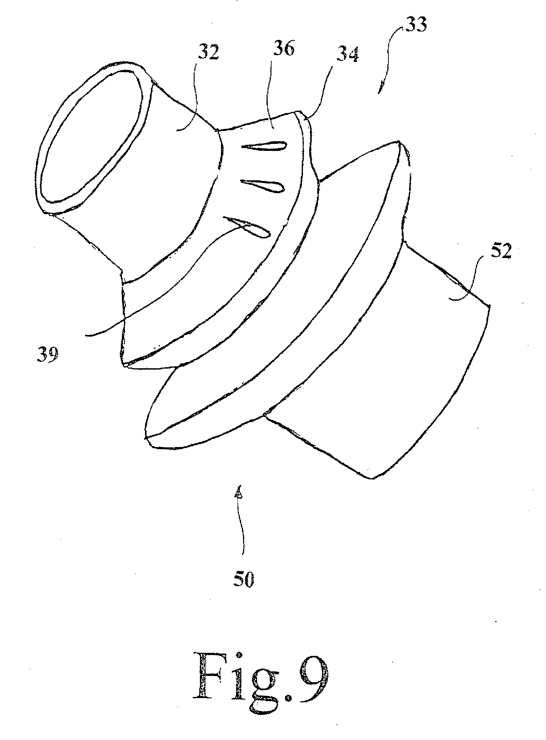

In Fig. 9 ist eine weitere Ausführungsform einer Ableitungseinrichtung 50 zur Ableitung von zumindest teilweise verbrauchtem Atemgas dargesteilt. Die Ableitungseinrichtung 50 umfaßt hier eine zum Anschluß einer Atemgasleitung vorgesehene Anschlußeinrichtung 32 sowie eine auf einer die Anschlußeinrichtung 32 abgewandten Seite ausgebildeten Anschlußstruktur 52, über welche die Ableitungseinrichtung mit einem Maskenbasiskörper 31 oder einem weiteren Atemgasleitungsabschnit koppelbar ist. Zwischen der Anschlußstruktur 52 und der Anschlußeinrichtung 32 ist eine Entkoppelungsstruktur 33 vorgesehen, durch welche eine Relativbewegung zwischen Anschlußeinrichtung 32 und Anschlußstruktur 52 in einem bestimmten Bewegungsbereich zugelassen ist. Bei der hier gezeigten Ausführungsform sind in einem Bewegungsbereich von bis zu 10 mm Axialbewegungen und in einem Winkelbereich von ca. 30° Kippbewegungen zugelassen in einem Umfangsabschnitt einer Umfangswandung 36 des ersten Faltenkranzes 34 sind, ähnlich wie bei der vorangehend beschriebenen Ausführungsform Atemgasdurchgangsöffnungen 39 ausgebildet. Die Atemgasdurchgangsöffnungen 39 sind hier als schmale im wesentlichen radial ausgerichtete Schlitze ausgebildet. Die Wanddicken der Umfangswandungen der beiden Faltenkränze 34 sind im Bereich der Knickstellen dünner gewählt, als in dem sich zwischen den Knickstellen erstreckenden Wandungsbereich. Die hier dargestellte Ausführungsform einer Ableitungseinrichtung weist im Bereich der Anschlußeinrichtung 32 einen Innendurchmesser von 18 mm und im Bereich der Entkoppelungsstruktur 33 einen Außendurchmesser von 35 mm auf. Die axiale Länge der Ableitungseinrichtung 50 beträgt im unbelasteten Zustand 54 mm. Die Ableitungseinrichtung 50 ist aus einem Elastomermaterial - hier volltransparenter Silikonkautschuk - gebildet. Die maximale Wanddicke der Ableitungseinrichtung 50 beträgt 4 mm.

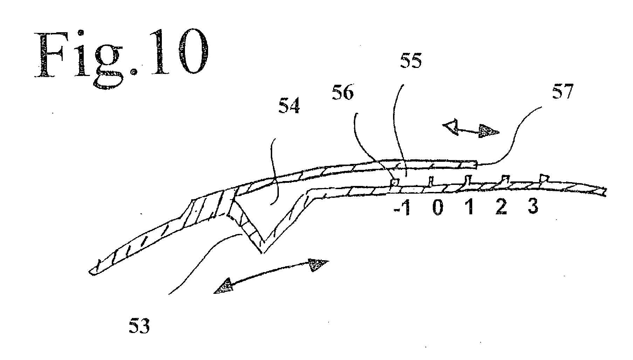

In Fig. 10 ist skizzenartig eine bevorzugte Ausführungsform einer Kanaleinrichtung zur Ableitung von Atemgas dargestellt. Der hier definierte Atemgasweg verläuft durch eine Durchgangsöffnung 53 in einem ersten Zwischenraum 54, der über einen Spaltbereich 55 mit der Umgebung in Verbindung steht. In diesem Spaltbereich sind mehrere Stegabschnitte 56 ausgebildet, durch welche die Drosselcharakteristik des Spaltbereiches beeinflußt wird. Anhand der Länge des Spaltbereiches 55 kann insbesondere im Zusammenspiel mit den Stegen 56 das Strömungsverhalten des Atemgasweges definiert beeinflußt werden. Ein derartiger Atemgasweg zur Ableitung von Atemgas aus einem Maskeninnenbereich in die Umgebung kann unmittelbar an einen Maskenbasiskörper 31 oder vorzugsweise im Bereich einer Anschlußeinrichtung zum Anschluß einer Atemgasleitung ausgebildet sein. Die hier gezeigte Struktur ist vorzugsweise integral aus einem vollelastomeren Material gebildet. Zum Zwecke der Reinigung ist es möglich, eine obere Deckwandung 57 nach oben aufzustülpen. Ein erforderliches Mindestspaltmaß in dem Spaltbereich 55 kann durch hier nicht dargestellte Stegabschnitte erreicht werden, die sich abschnittsweise bis an die Innenfläche der Deckenwandung 57 erstrecken.

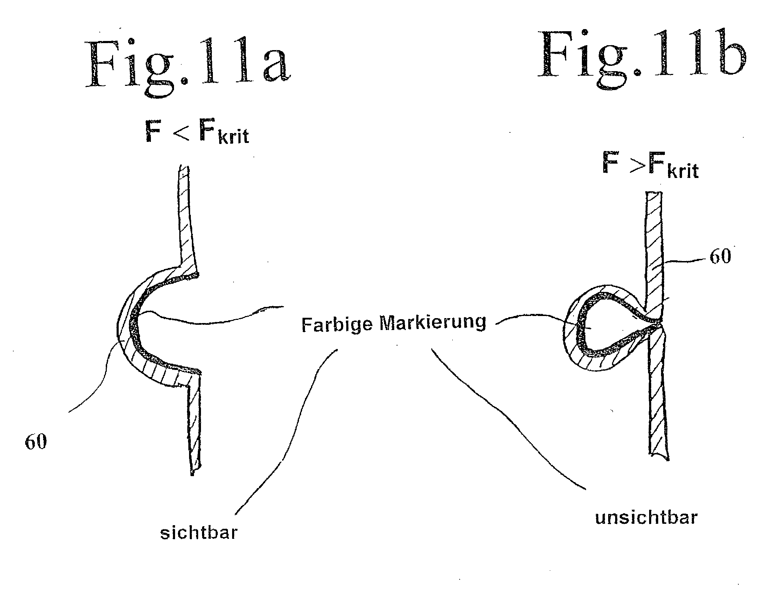

In Fig. 11 ist eine Elastomerstruktur dargestellt, die in Verbindung mit einer Atemmaske unmittelbar zur Anzeige der bei Applikation der Atemmaske stattfindenden Verformung geeignet ist. So ist es beispielsweise möglich, im Bereich der Innenwandung einer Falten- oder Balgstruktur 60 beispielsweise eine Farbmarkierung vorzusehen, die in Abhängigkeit von der Verformung der Falten- oder Balgstruktur erkennbar ist. Wird beispielsweise die Falten- oder Balgstruktur 60, wie in Fig. 11 dargestellt, maximal gestaucht, so ist die im Bereich der Innenwandung der Falten- oder Balgstruktur angebrachte Farbmarkierung von außen nicht mehr sichtbar. Hieraus kann beispielsweise auf eine unzulässig hohe Flächenpressung im Bereich der Stirnauflage und/oder im Bereich der Dichtlippeneinrichtung einer entsprechend ausgestalteten Atemmaske geschlossen werden. Es ist auch möglich, anhand einer derartigen Falten- oder Balgstruktur 60 zu überprüfen, ob eine ausreichende Maskenhaltekraft auf die Atemmaske aufgebracht ist. Bei einer derartigen Ausführungsform kann beispielsweise die farbliche Markierung derart angeordnet sein, daß diese im Falle unzureichender Maskenhaltekräfte sichtbar ist und erste bei ausreichenden Maskenhaltekräften abgedeckt ist:

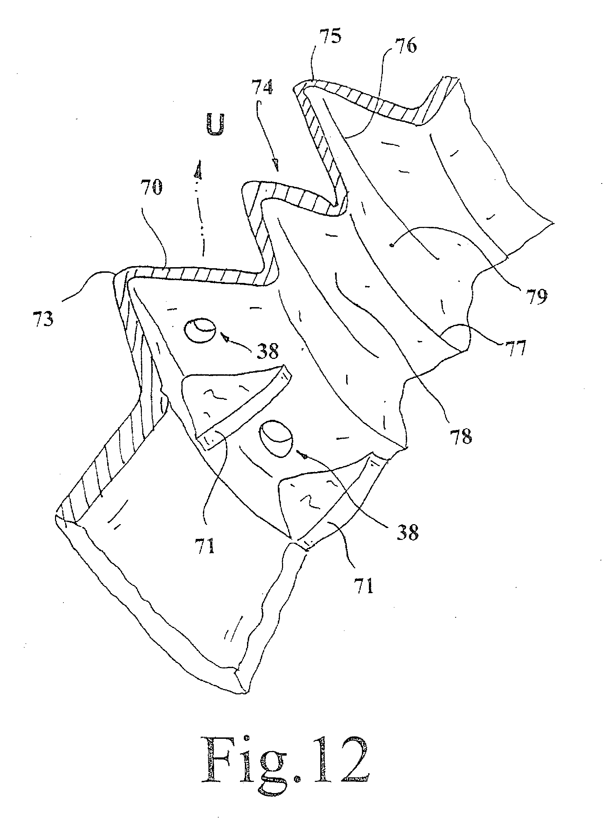

In Fig. 12 ist ein Abschnitt einer Ableitungseinrichtung zur Ableitung von Atemgas dargestellt, die aus einem Elastomermaterial gebildet ist. Die Ableitungseinrichtung umfaßt eine Umfangswandung 70, die hier als Faltenkranz 73 ausgebildet ist. Die Umfangswandung ist mit mehreren Durchgangsöffnungen 38 versehen zur Ableitung von Atemgas. Im Inneren des Faltenkranzes 73 sind Stege 71 ausgebildet, die integral mit der Umfangswandung 70 gefertigt sind. Die Stege 71 wirken als Knicksicherung und stellen dabei sicher, daß die Durchgangsöffnungen 38 ständig geöffnet sind. Durch die Stege 71 wird weiterhin eine Aufteilung der Gasströmung erreicht, wodurch sich eine noch geräuschärmere Ableitung des Atemgases in die Umgebung (U) ergibt. Die Durchgangsöffnungen weisen hier einen kreisrunden Querschnitt auf. Im Anschluß an den mit den Durchgangsöffnungen 38 versehenen Faltenkranz 73 schließt sich ein weiterer Faltenkranz 74 an. Dieser Faltenkranz 74 ist vergleichsweise biegesteif mit dem ersten Faltenkranz 73 gekoppelt und weist zudem eine geringere Höhe auf, so daß ein Abdekken der Durchgangsöffnung 38 durch den weiteren Faltenkranz 74 nicht möglich ist. An den weiteren Faltenkranz 74 schließt sich ein Gelenkfaltenkranz 75 an, der im Bereich seines maximalen Durchmessers eine Umfangsgelenkzone 76 und im Innenbereich eine Innengelenkzone 77 definiert. Im Bereich der Innengelenkzone 77 und der Umfangsgelenkzone 76 ist die Wanddicke der Umfangswandung 70 derart gering ausgebildet, daß eine vergleichsweise leichtgängige Bewegbarkeit der einander abgewandten Abschnitte der Ableitungseinrichtung zueinander gegeben ist. Die Umfangswandungen 78 des Faltenkranzes 74 und die Umfangswandung 79 des Gelenkfaltenkranzes 75 begrenzen gemeinsam den maximalen Schwenkwinkel der beiden Faltenkränze zueinander.

Die Funktionsweise der vorangehend beschriebenen Atemmaske wird anhand des nachfolgenden Anwendungsbeispieles im einzelnen erläutert:
Zur Durchführung einer CPAP-Therapie wird die Atemmaske einer Sterilverpakkung entnommen, und das Fixierelement 10 in eine Lösestellung geschwenkt, so daß der Versteifungsabschnitt 6b gegenüber dem ersten Versteifungsabschnitt 6a um die Filmscharnierstelle 22 schwenkbewegbar ist. Nunmehr wird an die Atemmaske über einen Dreh- und Schnellverschlußadanter ein Atemgasschlauch angeschlossen, in dem dieser auf den Schnellverschlußadapter und dieser in den Atemschlauchanschlußadapter 20 eingesteckt wird. Über den Atemgasschlauch wird Atemgas unter einem vorbestimmten Überdruck von beispielsweise 8 mbar zugeführt. Nunmehr wird die Atemmaske auf dem Nasenbereich des Maskenanwenders appliziert. Hierzu wird durch die unteren Koppelungseinrichtungen 15 der jeweilige Abschnitt einer unteren Kopfbandanordnung hindurchgeführt. Die Zugspannung in der unteren Kopfbandanordnung wird derart eingestellt, bis eine ausreichende Dichtigkeit der Dichtlippeneinrichtung 2 gewährleistet ist. Nunmehr wird die Atemmaske in Applikationsposition derart vom Nasenrücken weg, bzw. zum Nasenrücken hin gekippt, bis eine optimale Anlage der Dichtlippeneinrichtung 2 im Bereich des Nasenrückens erreicht wird. Nunmehr wird die obere Stirnpolstereinrichtung 4 leicht gegen die Stirn des Maskenanwenders gedrängt. Die hierbei erreichte Relativpositionierung des ersten Versteifungsabschnittes 6a relativ zu dem zweiten Versteifungsabschnitt 6b wird fixiert, indem das Fixierelement 10 mit dem Halteelement 11 in Eingriff gebracht wird.

Infolge des in der Atemgasleitung herrschenden Überdruckes strömt CO₂ durch die in dem Faltenkranz ausgebildeten Durchgangsöffnungen. Die Durchgangsöffnungen sind derart dimensioniert und ausgebildet, daß eine vorbestimmte Druck/Volumenstromcharakteristik erreicht wird, so daß ein ausreichender Abstrom der in die Atemmaske oder in die Atemgasleitung hinein exhalierten Atemluft in die Umgebung erreicht wird.

Durch die in dem Faltenkranz ausgebildeten Stege ist auf zuverlässige Weise sichergestellt, daß die Öffnungen infolge einer durch die Entkoppelungsstruktur zugelassenen Ausgleichsbewegung nicht verschlossen werden.

Die Atemmaske ist nunmehr gebrauchsfertig justiert. Durch Koppelung der Koppelungseinrichtung 16 im Bereich der Stirnauflageeinrichtung 3 wird nunmehr die Atemmaske auch im Stirnbereich des Maskenanwenders durch die obere Kopfbandanordnung am Maskenanwender fixiert.

Es ist möglich, die hier erreichte Relativpositionierung durch zusätzliche Maßnahmen beispielsweise ein in einrastender Weise blockierendes Element (z.B. Sicherungselement) zu fixieren. Bei entsprechender Tragfähigkeit dieser zusätzlichen Fixiermittel ist es möglich, das Fixierelement 10 und ggf. auch das Halteelement 11 zu entfernen.

Es ist auch möglich, anhand der über die Fixiereinrichtung 9 ermittelten idealen Konfiguration der Atemmaske ein Versteifungselement aus einem Versteifungselemente-Set auszuwählen, das unter Verzicht auf die Fixiereinrichtung 9 der Dichtlippeneinrichtung und der Stirnpolstereinrichtung 4 die gewünschte Relativposition verleiht - und ein derartiges Versteifungselement gegen das vorangehend genannte Versteifungselement auszutauschen.

## Patentansprüche

1. Atemmaske zur Zufuhr eines Atemgases zu einer Person mit: einer Dichtlippeneinrichtung (2) zur Abdichtung einer Maskenauflagezone, einem Maskenbasiskörper (1, 31) zur Bildung eines Maskeninnenraumes und einer Stirnauflageeinrichtung (3, 40) mit einem Polsterorgan, wobei die Dichtlippeneinrichtung(2), der Maskenbasiskörper (1, 31) und das Polsterorgan integral ausgebildet sind, **dadurch gekennzeichnet, dass** die Atemmaske ferner einen Verbindungsstegabschnitt (5) aufweist, der die Stirnauftageeinrichtung (3, 40) mit dem Maskenbasiskörper (1, 31) verbindet, wobei die Position der Stirnauflageeinrichtung (3, 40) und des Maskenbasiskörpers (1,31) zueinander veränderbar ist.

2. Atemmaske nach Anspruch 1, ferner mit einem Versteifungselement (6), das im Umfangsbereich der Maske vorgesehen ist.

3. Atemmaske nach Anspruch 2, wobei das Versteifungselement (6) einen der Umfangskontur der Dichtlippeneinrichtung (2) folgenden ersten Versteifungsabschnitt (6a) aufweist.

4. Atemmaske nach Anspruch 3, wobei das Versteifungselement (6) einen zweiten Versteifungsabschnitt (6b) aufweist, der sich in die Stirnauflageeinrichtung (3, 40) erstreckt.

5. Atemmaske nach Anspruch 4, wobei der erste Versteifungsabschnitt (6a) derart mit dem zweiten Versteifungsabschnitt (6b) verbunden ist, dass eine Bewegung jedes Versteifungsabschnitts (6a, 6b) bezügliche dem anderen Versteifungsabschnitt (6b, 6a) möglich ist.

6. Atemmaske nach Anspruch 5, wobei die beiden Versteifungsabschnitte (6a, 6b) über eine Gelenkeinrichtung (7) miteinander gekoppelt sind.

7. Atemmaske nach einem der Ansprüche 2 bis 6, wobei das Versteifungselement (6) mit dem Maskenbasiskörper (1, 31) und der Dichtlippeneinrichtung (2) derart gekoppelt ist, dass dieses die Gestalt des Maskenbasiskörper (1, 31) und der Dichtlippeneinrichtung (2) mitbestimmt.

8. Atemmaske nach einem der Ansprüche 2 bis 7, wobei der Maskenbasiskörper (1,31) eine Umfangsnut (14) aufweist, in die der erste Versteifungsabschnitt (6a) eingesetzt ist.

9. Atemmaske nach einem der Ansprüche 2 bis 8, wobei die Stirnauflageeinrichtung (3, 40) eine Stirnpolstereinrichtung (4) mit einer Nut aufweist, in die der zweite Versteifungsabschnitt (6b) eingesetzt ist.

10. Atemmaske nach einem der Ansprüche 2 bis 9, wobei das Versteifungselement (6) ferner eine erste Koppelungseinrichtung (15) zum Koppeln eines Bandelements eines Kopfbands an den Maskenbasiskörper (1, 31) aufweist.

11. Atemmaske nach einem der Ansprüche 2 bis 10, wobei das Versteifungselement (6) ferner eine zweite Koppelungseinrichtung (16) zum Koppeln eines Bandelements eines Kopfbands an die Stirnauflageeinrichtung (3, 40) aufweist.

12. Atemmaske nach einem der Ansprüche 10 oder 11, wobei die erste und/oder die zweite Koppelungseinrichtung (15, 16) integral mit dem Versteifungselement (6) ausgebildet ist/sind.

13. Atemmaske nach einem der Ansprüche 1 bis 12, wobei eine Fixiereinrichtung (9) vorgesehen ist, zur Fixierung der Stirnauflageeinrichtungen (3, 40) relativ zum Maskenbasiskörper (1, 31) und/oder der Dichtlippeneinrichtung (2).

14. Atemmaske nach Anspruch 13, wobei die Fixiereinrichtung (9) ein Fixierelement (10) aufweist, das ausgebildet ist mit einem Haltelement (11) in einer von mehreren unterschiedlichen Kopplungspositionen in Eingriff zu kommen.

15. Atemmaske nach Anspruch 14, wobei das Fixierelement (9) mehrere Rastbohrungen(26, 27, 28, 29) aufweist und wobei das Haltelement (11) einen Fixierzapfen (24) aufweist, der ausgebildet ist, mit mindestens einer der mehreren Rastbohrungen (26, 27, 28, 29) in Eingriff zu kommen.

16. Atemmaske nach einem der Ansprüche 13 bis 15, wobei das Halteelement (11) im Wesentlichen biegesteif mit dem ersten Versteifungsabschnitt (6a) verbunden ist und das Fixierelement (9) schwenkbar mit dem zweiten Versteifungsabschnitt (6b) verbunden ist.

17. Atemmaske nach einem der Ansprüche 1 bis 16, wobei die Dichtlippeneinrichtung (2) und/oder die Stirnauflageeinrichtung (3, 40) aus einem elastomeren Material gebildeten ist/sind.

## Claims

1. A breathing mask for feeding a breathing gas to a person comprising: sealing lip means (2) for sealing a mask rest zone, a mask base body (1, 31) to form a mask interior space, and a forehead rest means (3, 40) having a padding member, wherein the sealing lip means (2), the mask base body (1, 31), and the padding member are formed integrally, **characterized in that** the breathing mask further comprises a connection web section (5) connecting the forehead rest means (3, 40) to the mask base body (1, 31), wherein the position of the forehead rest means (3, 40) and the mask base body (1, 31) is adjustable relative to each other.

2. The breathing mask according to claim 1, further comprising a reinforcement element (6), which is provided in the peripheral region of the mask.

3. A breathing mask according to claim 2, wherein the reinforcement element (6) comprises a first reinforcement section (6a) following the circumferential contour of the sealing lip means (2).

4. The breathing mask according to claim 3, wherein the reinforcement element (6) comprises a second reinforcement section (6b) extending into the forehead rest means (3, 40).

5. The breathing mask according to claim 4, wherein said first reinforcement section (6a) is connected to the second reinforcement section (6b) in such a way that movement of each reinforcement section (6a, 6b) relative to the other reinforcement section (6b, 6a) is admitted.

6. The breathing mask according to claim 5, wherein the two reinforcement sections (6a, 6b) are coupled to each other via a hinge means (7).

7. The breathing mask according to any one of claims 2 to 6, wherein the reinforcement element (6) is coupled to the mask base body (1, 31) and the sealing lip means (2) in such a way that it cooperates in determining the shape of the mask base body (1, 31) and the sealing lip means (2).

8. The breathing mask according to any one of claims 2 to 7, wherein the mask base body (1, 31) has a circumferential groove (14) into which the first reinforcement section (6a) is inserted.

9. The breathing mask according to any one of claims 2 to 8, wherein the forehead rest means (3, 40) includes a forehead padding means (4) having a groove into which the second reinforcement section (6b) is inserted.

10. The breathing mask according to any one of claims 2 to 9, wherein the reinforcement element (6) further comprises a first coupling means (15) for coupling a band element of a head band to the mask base body (1, 31).

11. The breathing mask according to any one of claims 2 to 10, wherein the reinforcement element (6) further comprises a second coupling means (16) for coupling a band element of a head band to the forehead rest means (3, 40).

12. The breathing mask according to any one of claims 10 or 11, wherein the first and/or second coupling means (15, 16) is/are formed integrally with the reinforcement element (6).

13. The breathing mask according to any one of claims 1 to 12, wherein a fixing means (9) is provided for fixing the forehead rest means (3, 40) relative to the mask base body (1,31) and/or the sealing lip means (2).

14. The breathing mask according to claim 13, wherein the fixing means (9) comprises a fixing element (10) formed to be engaged with a holding element (11) in one of a plurality of different coupling positions.

15. The breathing mask according to claim 14, wherein the fixing element (9) includes a plurality of catch bores (26, 27, 28, 29), and wherein the holding element (11) includes a fixing pin (24) formed to be engaged with at least one of the plurality of catch bores (26, 27, 28, 29).

16. The breathing mask according to any one of claims 13 to 15, wherein the holding element (11) is rigidly connected to the first reinforcement section (6a), and the fixing member (9) is pivotally connected to the second reinforcement section (6b).

17. The breathing mask according to any one of claims 1 to 16, wherein the sealing lip means (2) and/or the forehead rest means (3, 40) is/are formed of an elastomer material.

## Revendications

1. Masque respiratoire destiné à fournir un gaz respiratoire à une personne avec : un dispositif à lèvre d'étanchéité (2) pour rendre étanche une zone d'appui de masque, un corps de base de masque (1, 31) pour la formation d'un espace intérieur de masque et un dispositif d'appui frontal (3, 40) avec un organe de rembourrage, le dispositif à lèvre d'étanchéité (2), le corps de base de masque (1, 31) et l'organe de rembourrage étant réalisés d'un seul bloc, **caractérisé en ce que** le masque respiratoire présente en outre une section de nervure de liaison (5) qui relie le dispositif d'appui frontal (3, 40) au corps de base de masque (1, 31), les positions du dispositif d'appui frontal (3, 40) et du corps de base de masque (1, 31) étant modifiables l'une par rapport à l'autre.

2. Masque respiratoire selon la revendication 1, en outre avec un élément de renforcement (6) qui est prévu dans la zone périphérique du masque.

3. Masque respiratoire selon la revendication 2, l'élément de renforcement (6) comprenant une première section de renforcement (6a) suivant le contour périphérique du dispositif à lèvre d'étanchéité (2).

4. Masque respiratoire selon la revendication 3, l'élément de renforcement (6) comprenant une seconde section de renforcement (6b) qui s'étend dans le dispositif d'appui frontal (3, 40).

5. Masque respiratoire selon la revendication 4, la première section de renforcement (6a) étant reliée à la seconde section de renforcement (6b) de telle manière qu'un mouvement de chaque section de renforcement (6a, 6b) soit possible par rapport à l'autre section de renforcement (6b, 6a).

6. Masque respiratoire selon la revendication 5, les deux sections de renforcement (6a, 6b) étant couplées l'une à l'autre par un dispositif d'articulation (7).

7. Masque respiratoire selon l'une quelconque des revendications 2 à 6, l'élément de renforcement (6) étant couplé au corps de base de masque (1, 31) et au dispositif à lèvre d'étanchéité (2) de manière à déterminer la forme du corps de base de masque (1, 31) et du dispositif à lèvre d'étanchéité (2).

8. Masque respiratoire selon l'une quelconque des revendications 2 à 7, le corps de base de masque (1, 31) comprenant une rainure périphérique (14), dans laquelle la première section de renforcement (6a) est insérée.

9. Masque respiratoire selon l'une quelconque des revendications 2 à 8, le dispositif d'appui frontal (3, 40) comprenant un dispositif de rembourrage frontal (4) avec une rainure, dans laquelle la seconde section de renforcement (6b) est insérée.

10. Masque respiratoire selon l'une quelconque des revendications 2 à 9, l'élément de renforcement (6) comprenant en outre un premier dispositif de renforcement (15) pour le couplage d'un élément d'une bande de tête sur le corps de base de masque (1, 31).

11. Masque respiratoire selon l'une quelconque des revendications 2 à 10, l'élément de renforcement (6) comprenant en outre un second dispositif de couplage (16) pour le couplage d'un élément d'une bande de tête sur le dispositif d'appui frontal (3, 40).

12. Masque respiratoire selon l'une quelconque des revendications 10 ou 11, le premier et/ou le second dispositif de couplage (15, 16) étant réalisés d'un seul bloc avec l'élément de renforcement (6).

13. Masque respiratoire selon l'une quelconque des revendications 1 à 12, un dispositif de fixation (9) étant prévu pour la fixation des dispositifs d'appui frontal (3, 40) par rapport au corps de base de masque (1, 31) et/ou au dispositif à lèvre d'étanchéité (2).

14. Masque respiratoire selon la revendication 13, le dispositif de fixation (9) comprenant un élément de fixation (10) qui est réalisé afin de venir en prise avec un élément de retenue (11) dans l'une des plusieurs positions de couplage différentes.

15. Masque respiratoire selon la revendication 14, l'élément de fixation (9) comprenant plusieurs perçages d'encliquetage (26, 27, 28, 29) et l'élément de retenue (11) comprenant un tenon de fixation (24) qui est réalisé afin de venir en prise avec au moins l'un des plusieurs perçages d'encliquetage (26, 27, 28, 29).

16. Masque respiratoire selon l'une quelconque des revendications 13 à 15, l'élément de retenue (11) étant sensiblement relié de manière résistante à la flexion à la première section de renforcement (6a) et l'élément de fixation (9) étant relié de manière pivotante à la seconde section de renforcement (6b).

17. Masque respiratoire selon l'une quelconque des revendications 1 à 16, le dispositif à lèvre d'étanchéité (2) et/ou le dispositif d'appui frontal (3, 40) étant constitués d'un matériau élastomère.
